# EUROPEAN PATENT SPECIFICATION

(11) **EP 1 269 972 B1**
(45) Date of publication and mention of the grant of the patent: **19.10.2005**
(21) Application number: 01917831.8
(22) Date of filing: 05.04.2001
(51) Int. Cl.: A61K 7/06, A61P 17/14

(54) **HAIR GROWTH STIMULANT COMPOSITIONS WITH SUSTAINED ACTION**
HAARWACHSTUMSTIMULIERENDE MITTEL MIT VERLÄNGERTER WIRKUNG
COMPOSITIONS DE TONIQUES CAPILLAIRES A ACTION DURABLE

(30) Priority: 07.04.2000 JP 2000106498
(43) Date of publication of application: 02.01.2003
(73) Proprietor: TAISHO PHARMACEUTICAL CO., LTD, Tokyo 170-8633 (JP)
(72) Inventor: IMAMURA, Koji, Toshima-ku, Tokyo 170-8633 (JP); OCHIAI, Rumi, Toshima-ku, Tokyo 170-8633 (JP); OKAJIMA, Takako, Toshima-ku, Tokyo 170-8633 (JP); MORIOKA, Susumu, Toshima-ku, Tokyo 170-8633 (JP); HORIE, Taro, Toshima-ku, Tokyo 170-8633 (JP)
(74) Representative: HOFFMANN EITLE
(86) International application number: PCT/JP2001/002941
(87) International publication number: WO 2001/078662

(56) References cited:
- EP-A1- 0 970 683
- WO-A-99/53923
- JP-A- 6 211 681

## Description

### TECHNICAL FIELD

The present invention relates to a hair growth stimulant composition with sustained action (sustained release hair growth composition) comprising minoxidil as an active component, and more particularly to a sustained release hair growth composition possessing a prolonged effect achieved by retaining minoxidil in the dermis of the scalp for a long period of time.

### BACKGROUND ART

The chemical name for minoxidil is 6-(1-piperidinyl)-2,4-pyrimidinediamine-3-oxide. The adaptation of minoxidil as a hair growth agent is disclosed in USP No. 4,139,619. Due to the excellent hair growth obtained by the topical application of minoxidil, hair growth agents containing minoxidil (hereinafter referred to as "minoxidil preparations") have been widely accepted and their sales volume is record breaking.

Because minoxidil preparations have to be applied several times a day, application tends to be forgotten during busy times. As a result, there are instances where a sufficient hair growth effect is not obtained. Research has been conducted to obtain a sustained release minoxidil preparation having a sufficient hair growth effect through a once-per-day application to coincide with the rhythm of human life, however there are currently very few sustained release minoxidil preparations that can satisfactorily accomplish this.

Patent document EP-A-0 970 683 discloses a hair growth composition (ex. 2) containing 2% minoxidil, 15% dipropylene glycol and 5% 1,3-butylene glycol.

The present invention was accomplished in light of this situation to obtain a sustained release minoxidil preparation having an excellent hair growth effect through a once-per-day application.

### DISCLOSURE OF THE INVENTION

To investigate the conditions under which the percutaneously absorbed minoxidil was stably retained in the dermis of the scalp, the present inventors examined the composition of the minoxidil preparation. As a result, the inventors have discovered that the above objective can be accomplished using a solution of a specific dihydric alcohol and a specific trihydric alcohol, thereby completing the present invention.

Accordingly, an object of the present invention is to provide a sustained release hair growth composition comprising (A) 2 to 6% by mass of minoxidil and (B) 5 to 30% by mass of a mixture of 1,3-butylene glycol or dipropylene glycol with glycerol blended at a weight ratio of 10:1 to 1:4.

### BRIEF DESCRIPTION OF THE DRAWING

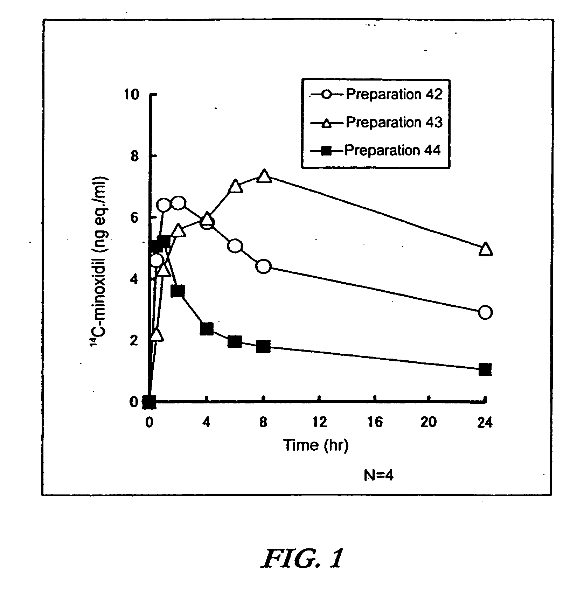

Figure 1 is a graph showing the measurement results of the concentration of minoxidil in the blood for the preparations of the present invention and a comparative preparation.

### DETAILED DESCRIPTION OF THE PREFERRED EMBODIMENT

The sustained release hair growth composition of the present invention is produced by mixing 5 to 30% by mass (hereinafter simply referred to as "%") of a mixture of 1,3-butylene glycol or dipropylene glycol with glycerol blended at a weight ratio of 10:1 to 1:4 (component (B)) and 2 to 6% of the active component minoxidil (component (A)), respectively for the total amount of the composition.

The sustained release hair growth composition even more preferably contains 3 to 6% of the component (A) (minoxidil). In view of sustained release action, the amount of the component (B) is preferably 10% or more and even more preferably 14% or more. In view of feeling during use, the amount is preferably 20% or less. Also, in view of the solubility of minoxidil and feeling during use of the hair growth composition, the incorporated amount of the component (B) (weight ratio; hereinafter the same) in regard to the component (A) is even more preferably in a range of about 1:1 to 1:5.

In the present invention, a mixture of 1,3-butylene glycol or dipropylene glycol as the dihydric alcohol and glycerol as the trihydric alcohol with a weight ratio of 10:1 to 1:4 must be used as the component (B). 1,3-butylene glycol or dipropylene glycol and glycerol at a weight ratio of 6:1 to 1:1 are even more preferable. The dihydric alcohol of the component (B) may be a mixture of 1,3-butylene glycol and dipropylene glycol.

The sustained release hair growth composition of the present invention is composed of the above composition, however, when necessary ethanol and water may be combined with the composition. In this instance, a preferable range for the incorporated amount of ethanol is about 40 to 82% and a preferable range for the incorporated amount of water is 5 to 30%.

The sustained release hair growth composition of the present invention is preferably adjusted to a pH of 5.5 to 9.5, and even more preferably to a pH of 5.5 to 6.5. An acid and/or base may be used as the pH adjustor for adjusting the pH. As preferable examples of the pH adjustor, citric acid, hydrochloric acid, lactic acid, phosphoric acid, and the like can be given.

In addition to the above components, other necessary active components and supplementary components may be added to the sustained release hair growth composition of the present invention. As medicinal components that are preferably added to and combined with the sustained release hair growth composition of the present invention, components selected from the group consisting of menthol, vitamin E acetate, pantothenylethylether, hinokitiol, glycyrrhetic acid, and diphenhydramine hydrochloride can be given (hereinafter referred to as "optional components"). Of these optional components, menthol and pantothenylethylether are preferable in view of sustained release action.

There are no particular limitations to the amount of these optional components to be added. This can be decided experimentally while taking the feeling during use and stability of minoxidil or solvent-type components into consideration. For example, the final composition preferably contains about 0.2 to 0.4% of menthol, a particularly preferable optional component.

In addition to the above components, the sustained release hair growth composition of the present invention may contain various active components and supplementary components normally used in topical preparations in an amount that does not harm the effect of the present invention. Examples of these components include fillers, vasodilators (carpronium chloride, benzyl nicotinate, swertia herb extract, panax ginseng extract, capsicum tincture, and the like), antihistamines (isothipendyl hydrochloride and the like), anti-inflammatory agents (guaiazulene and the like), keratolytics (urea, salicylic acid, and the like), antimicrobial agents (chlorhexidine gluconate, isopropyl methylphenol, quaternary ammonium salts, piroctone olamine, and the like), humectants (sodium hyaluronate, chondroitin sulfuric acid, and the like), extracts of animals and plants (Taxus cuspidata, Paeonia suffruticosa, Glycyrrhisa uralensis, Hypericum erectum, aconite root, Eriobotrya japonica, Artemisia capillaris, Symphytum officinale, Angelica keiskei, Crocus sativus, Gardeniae fructus, Rosmarinus officinalis, Salvia officinalis, Saussurea lappa, Aristolochia debilis, Lupuli strobilus, placenta, and the like), vitamins (retinol acetate, pyridoxine hydrochloride, ascorbic acid, thiamin nitrate, cyanocobalamin, biotin, and the like), anti-oxidants (dibutylhydroxytoluene, sodium pyrosulfite, tocopherol, sodium edetate, ascorbic acid, isopropyl gallate, and the like), solubilizers (diisopropyl adipate, isopropyl myristate, vegetable oils, animal oils, alkyl glyceryl ethers, hydrocarbons, and the like), metabolic activators, gelling agents (water-soluble high molecular compounds and the like), adhesives, perfumes, refrigerants (mentha oil, camphor, and the like), dyes, and the like.

However, since the addition of surfactants affects the cutaneous absorption of minoxidil and decreases the feeling during use, it is preferable that the sustained release hair growth composition of the present invention does not substantially contain surfactants.

The sustained release hair growth composition of the present invention thus obtained can be used as a suitable topical preparation such as a lotion, aerosol, tonic, cream, ointment, gel, and the like.

### EXAMPLES

The present invention will be described in more detail by examples, which should not be construed as limiting the present invention.

### Example 1

### Sustained release hair growth lotion:

2 g of minoxidil, 7 g of 1,3-butylene glycol, 3 g of glycerol, and 60.05 g of ethanol were mixed and stirred until dissolved. Next, purified water was added to the mixture to obtain 100 ml of a lotion (preparation 1).

### Example 2

Using the components shown in Tables 1-9, lotion preparations 2-38 (products of the present invention) were prepared in the same manner as in Example 1. Likewise, the lotion preparations 39-41 (comparative products) were prepared using the components shown in Table 10. Using the following test method, the concentration of minoxidil in the blood for the lotion products of the present invention and the comparative lotion products was measured. These results showed that minoxidil in the products of the present invention was released more slowly and retained for a longer period of time.

### (Composition)

**Table 1**

| | Preparation 1 | Preparation 2 | Preparation 3 |
|---|---|---|---|
| minoxidil | 2 g | 2 g | 2 g |
| 1,3-butylene glycol | 7 g | 6 g | 5 g |
| glycerol | 3 g | 4 g | 5 g |
| ethanol | 60.05 g | 60.05 g | 60.05 g |
| purified water | balance* | balance* | balance* |
| pH | 8.6 | 8.7 | 8.7 |

| | | | |
|---|---|---|---|
| *The total amount of the preparation is 100 ml | | | |

**Table 2**

| | Preparation 4 | Preparation 5 | Preparation 6 | Preparation 7 |
|---|---|---|---|---|
| minoxidil | 3 g | 3 g | 5 g | 5 g |
| 1,3-butylene glycol | 6 g | 5 g | 10 g | 15 g |
| glycerol | 4 g | 5 g | 10 g | 15g |
| ethanol | 60.05 g | 60.05 g | 51.47 g | 42.89 g |
| purified water | balance* | balance* | balance* | balance* |
| pH | 8.8 | 8.8 | 9.0 | 8.9 |

| | | | | |
|---|---|---|---|---|
| *The total amount of the preparation is 100 ml | | | | |

**Table 3**

| | Preparation 8 | Preparation 9 | Preparation 10 | Preparation 11 | Preparation 12 |
|---|---|---|---|---|---|
| minoxidil | 3 g | 3 g | 3 g | 5 g | 5 g |
| 1,3-butyiene glycol | 7 g | 6 g | 5 g | 17 g | 15 g |
| glycerol | 3 g | 4 g | 5 g | 3 g | 5 g |
| ethanol | 60.05 g | 60.05 g | 60.05 g | 51.47 g | 51.47 g |
| citric acid | proper amount | - | - | - | 1.0 g |
| phosphoric acid | - | proper amount | - | - | - |
| lactic acid | - | - | proper amount | - | - |
| hydrochloric acid | - | - | - | proper amount | - |
| sodium hydroxide | - | - | - | - | proper amount |
| purified water | balance* | balance* | balance* | balance* | balance* |
| pH | 6.0 | 6.0 | 5.5 | 5.6 | 6.5 |

| | | | | | |
|---|---|---|---|---|---|
| *The total amount of the preparation is 100 ml | | | | | |

**Table 4**

| | Preparation 13 | Preparation 14 | Preparation 15 | Preparation 16 | Preparation 17 |
|---|---|---|---|---|---|
| minoxidil | 5 g | 5 g | 5 g | 5 g | 5 g |
| 1,3-butylene glycol | 18 g | 15 g | 13 g | 12 g | 10 g |
| glycerol | 3 g | 5 g | 7 g | 8 g | 10 g |
| ethanol | 60.05 g | 60.05 g | 60.05 g | 60.05 g | 60.05 g |
| citric acid | proper amount | - | - | - | - |
| phosphoric acid | - | proper amount | - | - | - |
| lactic acid | - | - | proper amount | - | 1.0 g |
| hydrochloric acid | - | - | - | proper amount | - |
| sodium hydroxide | - | - | - | - | proper amount |
| purified water | balance* | balance* | balance* | balance* | balance* |
| pH | 6.0 | 6.0 | 5.5 | 5.6 | 6.5 |

| | | | | | |
|---|---|---|---|---|---|
| *The total amount of the preparation is 100 ml | | | | | |

**Table 5**

| | Preparation 18 | Preparation 19 | Preparation 20 |
|---|---|---|---|
| minoxidil | 2 g | 2 g | 2 g |
| dipropylene glycol | 7 g | 6 g | 5 g |
| glycerol | 3 g | 4 g | 5 g |
| ethanol | 60.05 g | 60.05 g | 60.05 g |
| purified water | balance* | balance* | balance* |
| pH | 8.6 | 8.7 | 8.6 |

| | | | |
|---|---|---|---|
| *The total amount of the preparation is 100 ml | | | |

**Table 6**

| | Preparation 21 | Preparation 22 | Preparation 23 | Preparation 24 |
|---|---|---|---|---|
| minoxidil | 3 g | 3 g | 5 g | 5 g |
| dipropylene glycol | 6 g | 5 g | 10 g | 15 g |
| glycerol | 4 g | 5 g | 10 g | 15g |
| ethanol | 60.05 g | 60.05 g | 51.47 g | 42.89 g |
| purified water | balance* | balance* | balance* | balance* |
| pH | 8.8 | 8.8 | 8.9 | 9.0 |

| | | | | |
|---|---|---|---|---|
| *The total amount of the preparation is 100 ml | | | | |

**Table 7**

| | Preparation 25 | Preparation 26 | Preparation 27 | Preparation 28 | Preparation 29 |
|---|---|---|---|---|---|
| minoxidil | 3 g | 3 g | 3 g | 5 g | 5 g |
| dipropylene glycol | 7 g | 6 g | 5 g | 17 g | 15 g |
| glycerol | 3 g | 4 g | 5 g | 3 g | 5 g |
| ethanol | 60.05 g | 60.05 g | 60.05 g | 51.47 g | 51.47 g |
| citric acid | proper amount | - | - | - | 1.0 g |
| phosphoric acid | - | proper amount | - | - | - |
| lactic acid | - | - | proper amount | - | - |
| hydrochloric acid | - | - | - | proper amount | - |
| sodium hydroxide | - | - | - | - | proper amount |
| purified water | balance* | balance* | balance* | balance* | balance* |
| pH | 6.0 | 6.0 | 5.5 | 5.6 | 6.5 |

| | | | | | |
|---|---|---|---|---|---|
| *The total amount of the preparation is 100 ml | | | | | |

**Table 8**

| | Preparation 30 | Preparation 31 | Preparation 32 | Preparation 33 | Preparation 34 |
|---|---|---|---|---|---|
| minoxidil | 5 g | 5 g | 5 g | 5 g | 5 g |
| dipropylene glycol | 18 g | 15 g | 13 g | 12 g | 10 g |
| glycerol | 3 g | 5 g | 7 g | 8 g | 10 g |
| ethanol | 60.05 g | 60.05 g | 60.05 g | 60.05 g | 60.05 g |
| citric acid | proper amount | - | - | - | - |
| phosphoric acid | - | proper amount | - | - | - |
| lactic acid | - | - | proper amount | - | 1.0 g |
| hydrochloric acid | - | - | - | proper amount | - |
| sodium hydroxide | - - | | - | - | proper amount |
| purified water | balance* | balance* | balance* | balance* | balance* |
| pH | 6.0 | 6.0 | 5.5 | 5.6 | 6.5 |

| | | | | | |
|---|---|---|---|---|---|
| *The total amount of the preparation is 100 ml | | | | | |

**Table 9**

| | Preparation 35 | Preparation 36 | Preparation 37 | Preparation 38 |
|---|---|---|---|---|
| minoxidil | 5 g | 5 g | 5 g | 5 g |
| dipropylene glycol | 18 g | 15 g | 10 g | 12 g |
| glycerol | 3g | 5g | 5g | 6g |
| ethanol | 60.05 g | 60.05 g | 60.05 g | 60.05 g |
| citric acid | proper amount | - | - | - |
| phosphoric acid | - | - | proper amount | - - |
| lactic acid | - | - | - | proper amount |
| hydrochloric acid | - | 0.2 g | - | - |
| sodium hydroxide | - | proper amount | - | - |
| L-menthol | 0.3 g | 0.3 g | 0.3 g | 0.3 g |
| vitamin E acetate | 0.08 g | 0.08 g | - | - |
| pantothenylethylether | 1 g | 1 g | 1 g | 1 g |
| hinokitiol | 0.05 g | 0.05 g | - | - |
| glycyrrhetic acid | 0.1 g | 0.1 g | - | - |
| diphenhydramine hydrochloride | 0.1 g | 0.1 g | - | - |
| purified water | balance* | balance* | balance* | balance* |
| pH | 6.0 | 6.1 | 6.0 | 6.0 |

| | | | | |
|---|---|---|---|---|
| *The total amount of the preparation is 100 ml | | | | |

**Table 10**

| | Preparation 39 | Preparation 40 | Preparation 41 |
|---|---|---|---|
| minoxidil | 2 g | 3 g | 5 g |
| 1,3-butylene glycol | 10 g | 10 g | 10 g |
| glycerol | - | - | - |
| ethanol | 60.05 g | 60.05 g | 60.05 g |
| citric acid | - | - | proper amount |
| purified water | balance* | balance* | balance* |
| pH | 8.7 | 8.8 | 6.0 |

| | | | |
|---|---|---|---|
| *The total amount of the preparation is 100 ml | | | |

### Example 3

Using the components shown in Table 11, lotion preparations 42 and 43 (products of the present invention) were prepared in the same manner as in Example 1. Likewise, lotion preparation 44 (comparative product) was prepared using the components shown in Table 11. The results of measurement of the concentration of minoxidil in the blood for the lotion products of the present invention and the comparative lotion products using the following test method are shown in Figure 1. These results confirm that the products of the present invention release minoxidil much slower.

**Table 11**

| | Preparation 42 | Preparation 43 | Preparation 44 |
|---|---|---|---|
| minoxidil | 2 g | 2 g | 2 g |
| 1,3-butylene glycol | 8 g | - | 2 g |
| dipropylene glycol | - | 8 g | - |
| glycerol | 8 g | 8 g | 2 g |
| ethanol | 50 g | 50 g | 50 g |
| phosphoric acid | 0.1 ml | 0.1ml | - |
| purified water | balance* | balance* | balance* |
| pH | 6.0 | 6.0 | 8.7 |

| | | | |
|---|---|---|---|
| *The total amount of the preparation is 100 ml | | | |

### (Test method)

Male Wistar rats (8 weeks old), whose abdominal hairs were shaved, were positioned supine under ether anesthesia, and 30 µl of each lotion prepared with ¹⁴C-minoxidil was applied on the defined area (2 X 3 cm) of the abdomen. At each defined time period (0.5, 1, 2, 4, 6, 8, and 24 hours), blood was collected from the jugular vein, and the concentration of minoxidil in the blood was measured using a liquid scintillation counter.

### Example 4

An aerosol was prepared by filling an aerosol can with 30 ml of the lotion prepared in Example 1 and 70 ml of dimethyl ether. In the same manner, an aerosol was prepared using the lotion of Example 2.

## Claims

1. A sustained release hair growth composition comprising (A) 2 to 6% by mass of minoxidil and (B) 5 to 30% by mass of a mixture of 1,3-butylene glycol or dipropylene glycol with glycerol blended at a weight ratio of 10:1 to 1:4.

2. The sustained release hair growth composition according to Claim 1, wherein the weight ratio of 1,3-butylene glycol or dipropylene glycol and glycerol of the component (B) is 6:1 to 1:1.

3. The sustained release hair growth composition according to either Claim 1 or 2, wherein the weight ratio of the minoxidil of the component (A) and the mixed solution of the component (B) is 1:1 to 1:5.

4. The sustained release hair growth composition according to any one of Claims 1-3, wherein the content of the minoxidil of the component (A) is 3 to 6% by mass.

5. The sustained release hair growth composition according to any one of Claims 1-4, wherein the composition has a pH of 5.5 to 9.5.

6. The sustained release hair growth composition according to any one of Claims 1-4, wherein the composition has a pH of 5.5 to 6.5.

7. The sustained release hair growth composition according to any one of Claims 1-6 further comprising ethanol and water.

8. The sustained release hair growth composition according to Claim 7, wherein the content of ethanol is 40 to 82% by mass.

9. The sustained release hair growth composition according to either Claim 7 or 8, wherein the content of water is 5 to 30% by mass.

10. The sustained release hair growth composition according to any one of Claims 1-9 substantially not containing surfactants.

11. The sustained release hair growth composition according to any one of Claims 1-10 containing an acid and/or base as a pH adjustor.

12. The sustained release hair growth composition according to Claim 11, wherein the pH adjustor is citric acid, hydrochloric acid, lactic acid, or phosphoric acid.

13. The sustained release hair growth composition according to any one of Claims 1-12 further comprising one or more components selected from the group consisting of menthol and pantothenylethylether.

14. The sustained release hair growth composition according to any one of claims 1-13 in the form of a topical lotion.

15. The sustained release hair growth composition according to any one of claims 1-13 in the form of a topical aerosol.

16. The sustained release hair growth composition according to any one of claims 1-13 in the form of a gel.

17. A use of a hair growth composition comprising (A) 2-6 % by mass of minoxidil and (B) 1,3-butylene glycol or dipropylene glycol and glycerol for preparing a hair growth agent for sustaining the effect of the hair growth composition.

18. The use according to Claim 17, wherein the content of 1,3-butylene glycol or dipropylene glycol and glycerol is 5 to 30% by mass based on the hair growth composition.

19. The use according to either Claim 17 or 18, wherein the ratio of 1,3-butylene glycol or dipropylene glycol and glycerol is 10:1 to 1:4.

## Patentansprüche

1. Haarwachstumszusammensetzung mit verzögerter Freisetzung, umfassend (A) 2 bis 6 Massen-% Minoxidil und (B) 5 bis 30 Massen-% einer Mischung aus 1,3-Butylenglycol oder Dipropylenglycol mit zugemischtem Glycerin bei einem Gewichtsverhältnis von 10:1 bis 1:4.

2. Haarwachstumszusammensetzung mit verzögerter Freisetzung nach Anspruch 1, worin das Gewichtsverhältnis von 1,3-Butylenglycol oder Dipropylenglycol und Glycerin der Komponente (B) 6:1 bis 1:1 ist.

3. Haarwachstumszusammensetzung mit verzögerter Freisetzung nach Anspruch 1 oder 2, worin das Gewichtsverhältnis von Minoxidil der Komponente (A) und der gemischten Lösung der Komponente (B) 1:1 bis 1:5 ist.

4. Haarwachstumszusammensetzung mit verzögerter Freisetzung nach einem der Ansprüche 1 bis 3, worin der Gehalt an Minoxidil der Komponente (A) 3 bis 6 Massen-% ist.

5. Haarwachstumszusammensetzung mit verzögerter Freisetzung nach einem der Ansprüche 1 bis 4, worin die Zusammensetzung einen pH von 5,5 bis 9,5 hat.

6. Haarwachstumszusammensetzung mit verzögerter Freisetzung nach einem der Ansprüche 1 bis 4, worin die Zusammensetzung einen pH von 5,5 bis 6,5 hat.

7. Haarwachstumszusammensetzung mit verzögerter Freisetzung nach einem der Ansprüche 1 bis 6, weiterhin umfassend Ethanol und Wasser.

8. Haarwachstumszusammensetzung mit verzögerter Freisetzung nach Anspruch 7, worin der Gehalt an Ethanol 40 bis 82 Massen-% ist.

9. Haarwachstumszusammensetzung mit verzögerter Freisetzung nach einem der Ansprüche 7 oder 8, worin der Gehalt an Wasser 5 bis 30 Massen-% ist.

10. Haarwachstumszusammensetzung mit verzögerter Freisetzung nach einem der Ansprüche 1 bis 9, im wesentlichen ohne Tenside.

11. Haarwachstumszusammensetzung mit verzögerter Freisetzung nach einem der Ansprüche 1 bis 10, umfassend eine Säure und/oder Base als pH-Einstellmittel.

12. Haarwachstumszusammensetzung mit verzögerter Freisetzung nach Anspruch 11, worin das pH-Einstellmittel Zitronensäure, Salzsäure, Milchsäure oder Phosphorsäure ist.

13. Haarwachstumszusammensetzung mit verzögerter Freisetzung nach einem der Ansprüche 1 bis 12, weiterhin umfassend eine oder mehrere Komponenten, ausgewählt aus der Gruppe, bestehend aus Menthol und Pantothenylethylether.

14. Haarwachstumszusammensetzung mit verzögerter Freisetzung nach einem der Ansprüche 1 bis 13 in der Form einer topischen Lotion.

15. Haarwachstumszusammensetzung mit verzögerter Freisetzung nach einem der Ansprüche 1 bis 13 in Form eines topischen Aerosols.

16. Haarwachstumszusammensetzung mit verzögerter Freisetzung nach einem der Ansprüche 1 bis 13 in der Form eines Gels.

17. Verwendung einer Haarwachstumszusammensetzung, umfassend (A) 2 bis 6 Massen-% Minoxidil und (B) eine Mischung aus 1,3-Butylenglycol oder Dipropylenglycol und Glycerin, zur Erzeugung eines Haarwachstumsmittels zur Verzögerung der Wirkung der Haarwachstumszusammensetzung.

18. Verwendung nach Anspruch 17, worin der Gehalt an 1,3-Butylenglycol oder Dipropylenglycol und Glycerin 5 bis 30 Massen-%, bezogen auf die Haarwachstumszusammensetzung, ist.

19. Verwendung nach Anspruch 17 oder 18, worin das Verhältnis von 1,3-Butylenglycol oder Dipropylenglycol und Glycerin 10:1 bis 1:4 ist.

## Revendications

1. Composition pour la croissance des cheveux à libération soutenue comprenant (A) 2 à 6 % en masse de minoxidil et (B) 5 à 30 % en masse d'un mélange de 1,3-butylène glycol ou de dipropylène glycol avec du glycérol mélangés à un rapport en poids de 10 : 1 à 1 : 4.

2. Composition pour la croissance des cheveux à libération soutenue selon la revendication 1,dans laquelle le rapport en poids du 1,3-butylène glycol ou du dipropylène glycol et du glycérol du constituant (B) est de 6 : 1 à 1 : 1.

3. Composition pour la croissance des cheveux à libération soutenue selon l'une ou l'autre de la revendication 1 ou 2, dans laquelle le rapport en poids du minoxidil du constitutant (A) et de la solution mixte du constituant (B) est de 1 : 1 à 1 : 5.

4. Composition pour la croissance des cheveux à libération soutenue selon l'une quelconque des revendications 1 à 3, dans laquelle la teneur en minoxidil du constituant (A) est de 3 à 6 % en masse.

5. Composition pour la croissance des cheveux à libération soutenue selon l'une quelconque des revendications 1 à 4, dans laquelle la composition a un pH de 5,5 à 9,5.

6. Composition pour la croissance des cheveux à libération soutenue selon l'une quelconque des revendications 1 à 4, dans laquelle la composition a un pH de 5,5 à 6.5.

7. Composition pour la croissance des cheveux à libération soutenue selon l'une quelconque des revendications 1 à 6 comprenant en outre de l'éthanol et de l'eau.

8. Composition pour la croissance des cheveux à libération soutenue selon la revendication 7, dans laquelle la teneur en éthanol est de 40 à 82 % en masse.

9. Composition pour la croissance des cheveux à libération soutenue selon l'une ou l'autre de la revendication 7 ou 8, dans laquelle la teneur en eau est de 5 à 30 % en masse.

10. Composition pour la croissance des cheveux à libération soutenue selon l'une quelconque des revendications 1 à 9 ne contenant pratiquement pas de tensioactifs.

11. Composition pour la croissance des cheveux à libération soutenue selon l'une quelconque des revendications 1 à 10 contenant un acide et/ou une base comme ajusteur de pH.

12. Composition pour la croissance des cheveux à libération soutenue selon la revendication 11, dans laquelle l'ajusteur de pH est l'acide citrique, l'acide chlorhydrique, l'acide lactique, ou l'acide phosphorique.

13. Composition pour la croissance des cheveux à libération soutenue selon l'une quelconque des revendications 1 à 12 comprenant en outre un ou plusieurs constituants choisis dans le groupe constitué par le menthol et le pantothényléthyléther.

14. Composition pour la croissance des cheveux à libération soutenue selon l'une quelconque des revendications 1 à 13 sous la forme d'une lotion topique.

15. Composition pour la croissance des cheveux à libération soutenue selon l'une quelconque des revendications 1 à 13 sous la forme d'un aérosol topique.

16. Composition pour la croissance des cheveux à libération soutenue selon l'une quelconque des revendications 1 à 13 sous la forme d'un gel.

17. Utilisation d'une composition pour la croissance des cheveux comprenant (A) 2 à 6 % en masse de minoxidil et (B) du 1,3-butylène glycol ou du dipropylène glycol et du glycérol pour la préparation d'un agent pour la croissance des cheveux pour soutenir l'effet de la composition pour la croissance des cheveux.

18. Utilisation selon la revendication 17, dans laquelle la teneur en 1,3-butylène glycol ou en dipropylène glycol et en glycérol est de 5 à 30 % en masse sur la base de la composition pour la croissance des cheveux.

19. Utilisation selon l'une ou l'autre de la revendication 17 ou 18, dans laquelle le rapport du 1,3-butylène glycol ou du dipropylène glycol et du glycérol est de 10 : 1 à 1 : 4.
